# EUROPEAN PATENT APPLICATION

(11) **EP 4 717 148 A1**
(43) Date of publication of application: **01.04.2026**
(21) Application number: 25202732.1
(22) Date of filing: 17.09.2025
(51) Int. Cl.: A61B 3/00, A61B 3/10, A61B 3/103, A61B 3/107

(54) **EYE-EXAMINING APPARATUS FOR DIAGNOSING MYOPIA**

(30) Priority: 30.09.2024 KR 20240132863
(71) Applicant: Huvitz Co., Ltd., Anyang-si, Gyeonggi-do 14055 (KR)
(72) Inventor: JO, Sun Hyung, 14055 Anyang-si (KR); BEAK, Woo Kyung, 14055 Anyang-si (KR); JU, Ye Na, 14055 Anyang-si (KR)
(74) Representative: Henkel & Partner mbB

(57) **Abstract**

Disclosed is an eye-examining apparatus for diagnosing myopia capable of simultaneously measuring the corneal curvature, refractive power, and axial length of the eye to be examined. The eye-examining apparatus for diagnosing myopia includes a keratometer (10); a refractometer (30) including an infrared measurement light source (50) configured to emit an infrared measurement light for measuring a refractive power and an axial length of the eye to be examined (5), a lens array (44) configured to divide a signal light formed by the infrared measurement light being reflected off a retina of the eye to be examined (5) into multiple signal lights and focus the multiple signal lights, and a refractor sensor (46) configured to detect an image of the signal lights divided by the lens array (44); an axial length measurement unit (60) including a beam splitter (62) configured to divide the infrared measurement light emitted from the infrared measurement light source (50) into a reference light (R) and an infrared measurement light (L), a reference mirror (64) configured to reflect and transmit the reference light (R) divided by the beam splitter (20) back to the beam splitter (20) and change an optical path length (OPL) of the reference light according to a distance to the beam splitter (62), and a light detector (66) configured to detect an interference light (I) generated by superposition of a signal light (S) generated by the divided measurement light (L) being reflected off each layer of the eye to be examined (5) and the reference light (R) reflected by the reference mirror (64); and a computation unit (70) configured to calculate the corneal curvature, refractive power, and axial length of the eye to be examined (5).

## Description

### CROSS-REFERENCE TO RELATED APPLICATION

This application claims benefit of priority to Korean Patent Application No. 10-2024-0132863 filed on September 30, 2024, the entire contents of which are incorporated herein by reference.

### FIELD OF THE DISCLOSURE

The present disclosure relates to an eye-examining apparatus for diagnosing myopia, and more particularly, to an eye-examining apparatus for diagnosing myopia capable of simultaneously measuring the corneal curvature, refractive power, and axial length of the eye to be examined.

### BACKGROUND

An eye-examining apparatus is a device that measures the characteristics of the eye to be examined, such as the corneal curvature, refractive power, and astigmatism axis, in an objective manner by means of optical and electronic systems.

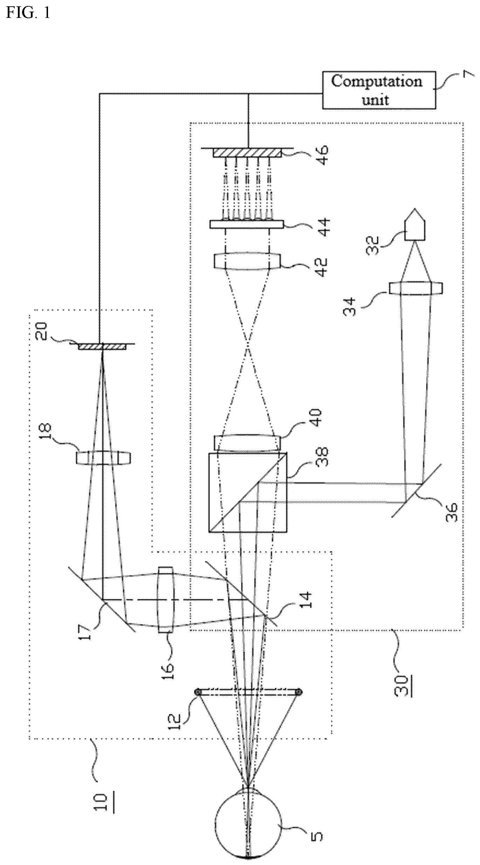

FIG. 1 is a diagram showing the configuration of a typical eye-examining apparatus. As shown in FIG. 1, the typical eye-examining apparatus consists of a keratometer 10 that measures the corneal curvature of the eye to be examined and a refractometer 30 that measures the refractive power of the eye to be examined. Looking into the operation of such an eye-examining apparatus, infrared light in the form of a mire ring is first emitted from a mire ring light source 12 of the keratometer 10 in order to measure the corneal curvature of the eye to be examined. The emitted infrared light is reflected off the cornea of the eye to be examined 5 and reflected again by a beam splitter 14, and then passes through a relay lens 16, an infrared reflective mirror 17, and an imaging lens 18, and the image of the mire ring reflected off the cornea of the eye to be examined 5 is formed on a keratometer sensor 20. A computation unit 7 calculates the corneal curvature of the eye to be examined 5 from the size, shape, etc., of the mire ring image detected by the keratometer sensor 20.

Next, the refractive power of the eye to be examined is measured using the refractometer 30. Specifically, a refractive power measurement light source 32 emits an infrared measurement light for measuring the refractive power of the eye to be examined 5, and the emitted measurement light passes through a collimation lens 34 that focuses the measurement light so that the focal point is formed on the retina of the eye to be examined 5, a reflective mirror 36, and a beam splitter 38 that reflects the measurement light to the eye to be examined 5, and focuses on the retina of the eye to be examined 5. The signal light formed by the measurement light being reflected off the retina of the eye to be examined 5 passes through the beam splitter 38, an objective lens 40 that focuses the signal light, an imaging lens 42 that converges the image of the focused signal light, and a lens array 44 that divides the converged signal light into multiple signal lights and focuses them. The image of the signal lights divided by the lens array 44 is formed on a refractor sensor 46. The computation unit 7 calculates the refractive power of the eye to be examined 5 from the image of the divided signal lights obtained from the refractor sensor 46.

On the other hand, in order to accurately diagnose the condition of the eye to be examined 5, specifically, whether it is myopic, it is necessary to measure not only the corneal curvature and refractive power of the eye to be examined 5 but also the distance from the cornea to the retina of the eye to be examined 5 (hereinafter referred to as "axial length"). However, typical eye-examining apparatuses can only measure the corneal curvature and refractive power of the eye to be examined 5 but not the axial length thereof. To measure the axial length of the eye to be examined, a separate axial length measurement device called a biometer must be used.

Therefore, in order to improve the efficiency of diagnosing myopia of the eye to be examined, there is a need for an eye-examining apparatus for diagnosing myopia capable of simultaneously measuring the corneal curvature, refractive power, and axial length of the eye to be examined.

### Prior Art Literature

Korean Patent Application Publication No. 10-2012-0090331

US Patent No. 7,884,946

### SUMMARY OF THE DISCLOSURE

### TECHNICAL OBJECTS

It is an object of the present disclosure to provide an eye-examining apparatus for diagnosing myopia capable of improving the efficiency of diagnosing myopia of an eye to be examined.

It is another object of the present disclosure to provide an eye-examining apparatus for diagnosing myopia capable of simultaneously measuring the corneal curvature, refractive power, and axial length of an eye to be examined.

### TECHNICAL SOLUTION

In order to achieve the above objects, the present disclosure provides an eye-examining apparatus for diagnosing myopia including a keratometer 10 including a mire ring light source 12 configured to emit a mire ring infrared light in the shape of a ring to a cornea of an eye to be examined 5, and a keratometer sensor 20 configured to detect a mire ring infrared light image reflected off the cornea of the eye to be examined 5; a refractometer 30 including an infrared measurement light source 50 configured to emit an infrared measurement light for measuring a refractive power and an axial length of the eye to be examined 5, a lens array 44 configured to divide a signal light formed by the infrared measurement light being refracted by the eye to be examined 5 and reflected off a retina of the eye to be examined 5 into multiple signal lights and focus the multiple signal lights, and a refractor sensor 46 configured to detect an image of the signal lights divided by the lens array 44; an axial length measurement unit 60 including a beam splitter 62 configured to divide the infrared measurement light emitted from the infrared measurement light source 50 into a reference light R and an infrared measurement light L, a reference mirror 64 configured to reflect and transmit the reference light R divided by the beam splitter 20 back to the beam splitter 20 and change an optical path length (OPL) of the reference light according to a distance to the beam splitter 62, and a light detector 66 configured to detect an interference light I generated by superposition of a signal light S generated by the divided measurement light L being reflected off each layer of the eye to be examined 5 and the reference light R reflected by the reference mirror 64; and a computation unit 70 configured to calculate a corneal curvature of the eye to be examined 5 from a size and shape of the mire ring image detected by the keratometer sensor 20, calculate a refractive power of the eye to be examined 5 from an image of the signal light detected by the refractor sensor 46, and calculate an axial length of the eye to be examined 5 from an intensity of the interference light I detected by the light detector 66 and a position of the reference mirror 64.

### EFFECTS OF THE DISCLOSURE

The eye-examining apparatus for diagnosing myopia according to the present disclosure can improve the efficiency of diagnosing myopia by simultaneously measuring the corneal curvature, refractive power, and axial length of an eye to be examined.

### BRIEF DESCRIPTION OF THE DRAWINGS

FIG. 1 is a diagram showing the configuration of a typical eye-examining apparatus; and
FIG. 2 is a diagram for describing the configuration of an eye-examining apparatus for diagnosing myopia according to one embodiment of the present disclosure.

### DETAILED DESCRIPTION OF THE PREFERRED EMBODIMENTS

Hereinafter, the present disclosure will be described in detail with reference to the accompanying drawings. In the accompanying drawings, elements that perform the same or similar functions as in the prior art are given the same reference numerals.

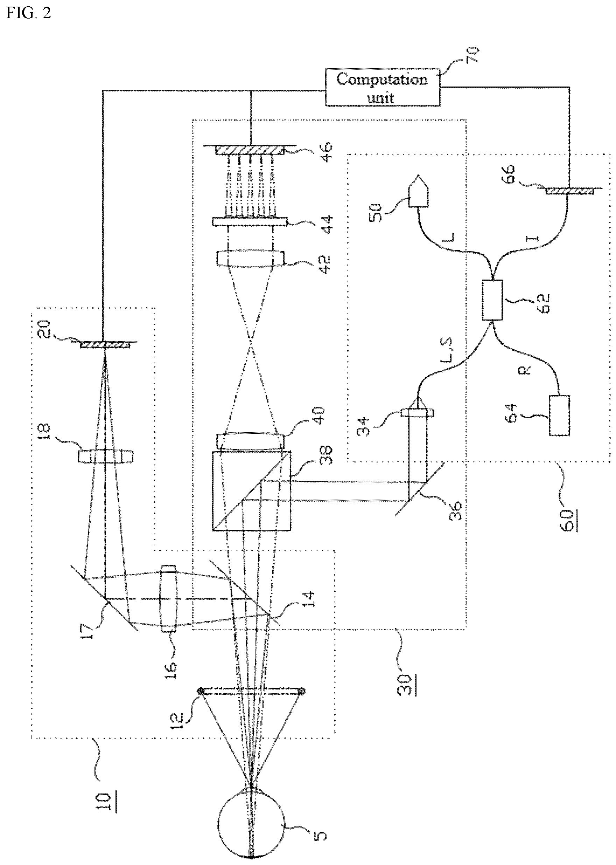

FIG. 2 is a diagram for describing the configuration of an eye-examining apparatus for diagnosing myopia according to one embodiment of the present disclosure. As shown in FIG. 2, the eye-examining apparatus for diagnosing myopia according to the present disclosure includes a keratometer 10 that measures the corneal curvature of an eye to be examined 5, a refractometer 30 that measures the refractive power, i.e., diopter, of the eye to be examined 5, an axial length measurement unit 60 that measures the axial length of the eye to be examined 5, and a computation unit 70.

The keratometer 10 includes a mire ring light source 12 that emits a mire ring infrared light in the shape of a ring to the cornea of the eye to be examined 5 and a keratometer sensor 20 that detects a mire ring infrared light image reflected off the cornea of the eye to be examined 5. The computation unit 70 calculates the corneal curvature of the eye to be examined 5 from the size and shape of the mire ring image detected by the keratometer sensor 20.

As the mire ring light source 12, a light-emitting diode (LED) in the infrared range may be used in order to suppress the pupillary reaction of the eye to be examined 5.

As needed, the keratometer 10 may further include a beam splitter 14 that separates the paths of the mire ring infrared light reflected off the cornea of the eye to be examined 5 and of an infrared measurement light emitted from an infrared measurement light source 50 of the refractometer 30 and the axial length measurement unit 60 described later, and guides the mire ring infrared light to the keratometer sensor 20. A dichroic mirror may be used as the beam splitter 14.

In addition, the keratometer 10 may further include a relay lens 16 that focuses and transmits the mire ring infrared light reflected off the cornea of the eye to be examined 5, an infrared reflective mirror 17 that changes the path of the mire ring infrared light reflected off the cornea of the eye to be examined 5, and an imaging lens 18 that focuses the mire ring infrared light reflected off the cornea of the eye to be examined 5 so that the focal point is formed on the keratometer sensor 20, allowing a clear mire ring infrared light image to be formed on the keratometer sensor 20, etc.

The refractometer (or refractor) 30 includes an infrared measurement light source 50 that emits an infrared measurement light for measuring the refractive power and axial length of the eye to be examined 5, a lens array 44 that divides a signal light formed by the infrared measurement light emitted from the infrared measurement light source 50 being refracted by the eye to be examined 5 and reflected off the retina of the eye to be examined 5 into multiple signal lights and focuses them, and a refractor sensor 46 that detects the image of the signal lights divided by the lens array 44.

The computation unit 70 obtains a wavefront topography map of the signal light wavefront from the image of the signal lights detected by the refractor sensor 46 and calculates the refractive power of the eye to be examined 5.

As described above, the refractometer 30 may share the beam splitter 14 with the keratometer 10 as necessary.

In addition, as needed, the refractometer 30 may further include a collimation lens 34 that causes the focal point of the infrared measurement light to be formed on the retina of the eye to be examined 5, a measurement light reflective mirror 36 that reflects the measurement light that has passed through the collimation lens 34, a polarizing beam splitter 38 that polarizes the reflected measurement light and reflects it to the eye to be examined 5, a focusing lens 40 that focuses the signal light formed by the measurement light that has been linearly polarized by the polarizing beam splitter 38 being reflected off the retina of the eye to be examined 5, an imaging lens 42 that converges the focused signal light, etc. The polarizing beam splitter 38 may be a 5:5 light splitting prism.

The axial length measurement unit 60 measures the axial length (length of the eye, distance from the cornea to the retina) of the eye to be examined 5 using the principle of an interferometer. As shown in FIG. 2, the axial length measurement unit 60 includes the infrared measurement light source 50, a beam splitter 62, a reference mirror 64, and a light detector 66.

The infrared measurement light source 50 is a device that emits an infrared measurement light for measuring the axial distance of the eye, and the infrared measurement light source 50 used in the refractometer 30 is also used here. In other words, the refractometer 30 and the axial length measurement unit 50 share the infrared measurement light source 50. The infrared measurement light used for measuring the axial length using an interferometer is typically a laser light having a short coherence distance, for example, a near-infrared laser light with a wavelength of 750 nm to 1500 nm. In the present disclosure, the infrared measurement light emitted from the infrared measurement light source 50 is used not only for measuring the axial length but also for measuring the refractive power of the eye to be examined 5, and is thus preferably a near-infrared measurement light with a wavelength of 800 to 880 nm, preferably 820 to 860 nm, for example, 830 or 850 nm.

A typical light-emitting diode (LED) or superluminescent diode (SLD) may be used as the infrared measurement light source 50.

The beam splitter 62 divides the infrared measurement light emitted from the infrared measurement light source 50 into a reference light R and an infrared measurement light L, and causes the reference light R to be irradiated onto the reference mirror 64 and the infrared measurement light L to be irradiated onto the eye to be examined 5. The beam splitter 62 may, for example, divide the infrared measurement light into an infrared reference light R and an infrared measurement light L with an intensity of 50:50. The reference light R divided by the beam splitter 62 is reflected by the reference mirror 64 and transmitted back to the beam splitter 62. The measurement light L divided by the beam splitter 62 and irradiated onto the eye to be examined 5 is reflected off each layer of the eye to be examined 5 and generates a signal light S. The generated signal light S and the reference light R reflected by the reference mirror 64 are superposed by the beam splitter 62 to generate an interference light I, and the generated interference light I is detected by the light detector 66. The beam splitter 62 also performs the role of superposing the reference light R and the signal light S and is thus also called a beam coupler.

The reference mirror 64 reflects the reference light R divided by the beam splitter 20 and transmits it back to the beam splitter 20, and changes the optical path length (OPL) of the reference light according to the distance to the beam splitter 62. For example, when the reference mirror 64 moves away from the beam splitter 62, the optical path length (OPL) of the reference light R increases. When the signal light S reflected off each layer of the eye to be examined 5 and the reference light R that has traveled the optical path of the same length are superposed, i.e., when the optical path lengths (OPLs) of the signal light S and the reference light R are identical, the signal light S and the reference light R interfere with each other and generate an interference light I signal.

The light detector 66 detects the interference light I generated by the superposition of the signal light S generated by the divided measurement light L being reflected off each layer of the eye to be examined 5 and the reference light R reflected by the reference mirror 64. An avalanche photodiode (APD) may be used as the light detector 66.

The computation unit 70 may calculate the position where the measurement light L is reflected off each layer of the eye to be examined 5, i.e., the distance from the cornea to the retina (axis length), from the intensity of the interference light I detected by the light detector 66 and the position of the reference mirror 64.

According to the eye-examining apparatus in accordance with the present disclosure, the refractive power of the eye to be examined can be calculated by detecting the signal light formed by the infrared measurement light emitted from the infrared measurement light source 50 being reflected off the retina of the eye to be examined 5 by the refractometer 30, and at the same time, the axial length of the eye to be examined 5 can be calculated by detecting the signal light by the axial length measurement unit 50. Further, the corneal curvature of the eye to be examined can be calculated together using the keratometer 10, as necessary. In other words, according to the present disclosure, early and precise diagnosis of myopia can be effectively performed by simultaneously measuring the corneal curvature, refractive power (diopter), and axial length of the eye to be examined.

Although the present disclosure has been described above with reference to the accompanying drawings and example embodiments, the present disclosure is not limited to what is shown in the drawings and the embodiments described above but should be construed to encompass all modifications, and equivalent constructions and functions of the example embodiments within the scope set forth in the following claims. Further, reference numerals are indicated in the following claims to facilitate understanding, but the scope of the following claims is not limited to what is shown by the reference numerals and in the drawings.

## Claims

1. An eye-examining apparatus for diagnosing myopia, comprising:
a keratometer (10) including a mire ring light source (12) configured to emit a mire ring infrared light in the shape of a ring to a cornea of an eye to be examined (5), and a keratometer sensor (20) configured to detect a mire ring infrared light image reflected off the cornea of the eye to be examined (5);
a refractometer (30) including an infrared measurement light source (50) configured to emit an infrared measurement light for measuring a refractive power and an axial length of the eye to be examined (5), a lens array (44) configured to divide a signal light formed by the infrared measurement light being refracted by the eye to be examined (5) and reflected off a retina of the eye to be examined (5) into multiple signal lights and focus the multiple signal lights, and a refractor sensor (46) configured to detect an image of the signal lights divided by the lens array (44);
an axial length measurement unit (60) including a beam splitter (62) configured to divide the infrared measurement light emitted from the infrared measurement light source (50) into a reference light (R) and an infrared measurement light (L), a reference mirror (64) configured to reflect and transmit the reference light (R) divided by the beam splitter (20) back to the beam splitter (20) and change an optical path length (OPL) of the reference light according to a distance to the beam splitter (62), and a light detector (66) configured to detect an interference light (I) generated by superposition of a signal light (S) generated by the divided measurement light (L) being reflected off each layer of the eye to be examined (5) and the reference light (R) reflected by the reference mirror (64); and
a computation unit (70) configured to calculate a corneal curvature of the eye to be examined (5) from a size and shape of the mire ring image detected by the keratometer sensor (20), calculate a refractive power of the eye to be examined (5) from an image of the signal light detected by the refractor sensor (46), and calculate an axial length of the eye to be examined (5) from an intensity of the interference light (I) detected by the light detector (66) and a position of the reference mirror (64).

2. The eye-examining apparatus for diagnosing myopia of claim 1, wherein the refractometer (30) and the axial length measurement unit (60) share the infrared measurement light source (50).

3. The eye-examining apparatus for diagnosing myopia of claim 1, wherein the infrared measurement light emitted from the infrared measurement light source (50) is a near-infrared measurement light with a wavelength of 800 to 880 nm.

4. The eye-examining apparatus for diagnosing myopia of claim 1, wherein the beam splitter (62) divides the infrared measurement light into an infrared reference light (R) and an infrared measurement light (L) with an intensity of 50:50.

5. The eye-examining apparatus for diagnosing myopia of claim 1, wherein the keratometer (10) and the refractometer (30) further comprise a beam splitter (14) configured to separate paths of the mire ring infrared light reflected off the cornea of the eye to be examined (5) and of the infrared measurement light emitted from the infrared measurement light source (50).
